# EUROPEAN PATENT APPLICATION

(11) **EP 4 242 315 A1**
(43) Date of publication of application: **13.09.2023**
(21) Application number: 21903560.7
(22) Date of filing: 12.07.2021
(51) Int. Cl.: C12N 15/77, C12N 9/50, C12P 13/06, C12P 13/08, C12R 1/15

(54) **MUTANT ATP-DEPENDENT PROTEASE, AND METHOD FOR PRODUCING L-AMINO ACID USING SAME**

(30) Priority: 11.12.2020 KR 20200173802
(71) Applicant: CJ CheilJedang Corporation, Seoul 04560 (KR)
(72) Inventor: YOON, Byoung, Hoon, Seoul 04560 (KR); KIM, Seon, Hye, Seoul 04560 (KR); BAE, Jee, Yeon, Seoul 04560 (KR); CHOI, Sun, Hyoung, Seoul 04560 (KR); KIM, Kyungrim, Seoul 04560 (KR); KIM, Hyung, Joon, Seoul 04560 (KR)
(74) Representative: Kuttenkeuler, David
(86) International application number: PCT/KR2021/008881
(87) International publication number: WO 2022/124511

(57) **Abstract**

Provided are an ATP-dependent protease variant and a method of producing L-amino acids using the same.

## Description

### BACKGROUND OF THE INVENTION

### 1. Field of the Invention

The present disclosure relates to an ATP-dependent protease variant and a method of producing L-amino acids using the same.

### 2. Description of the Related Art

L-Amino acids are basic structural blocks of proteins and have various important uses such as pharmaceutical raw materials and food additives, animal feeds, nutrients, pesticides, bactericides, etc. In particular, branched-chain amino acids (BCAA) collectively refer to L-valine, L-leucine, and L-isoleucine, which are essential amino acids, and the branched-chain amino acids are known to have an antioxidant effect and an effect of directly promoting protein synthesis in muscle cells.

Meanwhile, production of branched-chain amino acids using microorganisms is mainly carried out through microorganisms of the genus *Escherichia* or microorganisms of the genus *Corynebacterium,* and branched-chain amino acids are known to be biosynthesized from pyruvic acid via several steps using 2-ketoisocaproate as a precursor (U.S. Patent No. 9885093, U.S. Patent No. 10351859, U.S. Patent No. 8465962). However, the production of branched-chain amino acids through the microorganisms has a problem in that industrial mass-production is not easy.

However, with the increasing demand for branched-chain amino acids, research on the effective production capacity of branched-chain amino acids is still needed.

The present inventors have made intensive efforts to increase the production capacity of branched-chain amino acids, and as a result, they have developed a method of producing branched-chain amino acids at high concentrations using an ATP-dependent protease variant, thereby completing the present disclosure.

### SUMMARY OF THE INVENTION

An object of the present disclosure is to provide a microorganism of the genus *Corynebacterium,* in which ATP-dependent protease activity is weakened, as compared to an unmodified microorganism, preferably, a microorganism of the genus *Corynebacterium* having a branched-chain amino acid producing ability.

Another object of the present disclosure is to provide a ClpC variant, in which an amino acid sequence corresponding to positions 431 to 433 is deleted, based on an amino acid sequence represented by SEQ ID NO: 5.

Still another object of the present disclosure is to provide a polynucleotide encoding the ClpC variant of the present disclosure.

Still another object of the present disclosure is to provide a method of producing branched-chain amino acids, the method including the step of culturing, in a medium, the microorganism of the genus *Corynebacterium* of the present disclosure, or a microorganism of the genus *Corynebacterium* including any one or more of the ClpC variant of the present disclosure, or the polynucleotide of the present disclosure, and a vector including the same.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, those skilled in the art will recognize, or be able to ascertain using no more than routine experimentation, many equivalents to the specific embodiments of the disclosure described herein. Further, these equivalents should be interpreted to fall within the present disclosure.

An aspect of the present disclosure provides a microorganism of the genus *Corynebacterium,* in which ATP-dependent protease activity is weakened, as compared to an unmodified microorganism.

As used herein, the term "ATP-dependent protease (EC 3.4.21.92)" refers to an enzyme that hydrolyzes a protein into small peptides in the presence of ATP and Mg²⁺. The ATP-dependent protease of the present disclosure may be used interchangeably with endopeptidase Clp, ATP-dependent Clp protease, ClpP, or Clp protease.

Weakening of the ATP-dependent protease of the present disclosure may be weakening of activity of ATP-dependent Clp protease ATP-binding subunit.

The ATP-dependent Clp protease ATP-binding subunit may be used interchangeably with AAA family ATPase or ClpC. In the present disclosure, the sequence of the ATP-dependent Clp protease ATP-binding subunit may be obtained from GenBank of NCBI, which is a known database. Specifically, the subunit may be a polypeptide having the activity of ATP-dependent Clp protease ATP-binding subunit encoded by *clpC,* but is not limited thereto.

The ATP-dependent Clp protease ATP-binding subunit of the present disclosure may be derived from the genus *Corynebacterium.* For example, the ATP-dependent Clp protease ATP-binding subunit of the present disclosure may be derived from *Corynebacterium glutamicum.*

The ATP-dependent Clp protease ATP-binding subunit of the present disclosure may include a polypeptide represented by SEQ ID NO: 5 or an amino acid sequence having 90% or more identity thereto.

For example, the amino acid sequence having 90% or more identity to the amino acid sequence of SEQ ID NO: 5 of the present disclosure may be an amino acid sequence having at least 91% or more, 92% or more, 93% or more, 94% or more, 95% or more, 96% or more, 96.26% or more, 97% or more, 97.5% or more, 97.7% or more, 97.8% or more, 98% or more, 98.5% or more, 98.7% or more, 98.8% or more, 99% or more, 99.5% or more, 99.7% or more, 99.8% or more, or less than 100% homology or identity to the amino acid sequence of SEQ ID NO: 5 of the present disclosure. Further, it is apparent that proteins having amino acid sequences in which some sequences are deleted, modified, substituted, or added are also included within the scope of the protein to be weakened in the present disclosure as long as the amino acid sequences have such homology or identity and exhibit activity identical or corresponding to that of the ATP-dependent Clp protease ATP-binding subunit.

Further, the ATP-dependent Clp protease ATP-binding subunit of the present disclosure may have the polypeptide represented by the amino acid sequence of SEQ ID NO: 5 or the amino acid sequence having 90% or more identity thereto, or may consist of or may consist essentially of the polypeptide. The ATP-dependent Clp protease ATP-binding subunit of the present disclosure may include those having addition of a meaningless sequence upstream or downstream of SEQ ID NO: 5 or the amino acid sequence having 90% or more identity thereto (i.e., addition of a sequence that do not alter the function of the protein, at the N-terminus and/or C-terminus of the amino acid sequence), or a naturally occurring mutation, a silent mutation, or a conservative substitution.

The "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect activity of proteins or polypeptides.

The microorganism of the present disclosure may include a polypeptide having a deletion of the amino acids corresponding to positions 431 to 433, based on the sequence represented by SEQ ID NO: 5, or a polynucleotide having a deletion of nucleotides corresponding to positions 1,291 to 1,299, based on a sequence represented by SEQ ID NO: 6.

As used herein, the term "corresponding to" refers to amino acid residues or nucleotide residues at positions listed in the polypeptide or polynucleotide, or amino acid residues or nucleotide residues that are similar, identical, or homologous to those listed in the polypeptide or polynucleotide. Identifying the amino acid or nucleotide at the corresponding position may be determining a specific amino acid or nucleotide in a sequence that refers to a specific sequence. As used herein, "corresponding region" generally refers to a similar or corresponding position in a related protein or a reference protein.

For example, an arbitrary amino acid sequence is aligned with SEQ ID NO: 5 or SEQ ID NO: 6, and based on this, each amino acid residue of the amino acid sequence may be numbered with reference to the amino acid residue corresponding to the amino acid residue of SEQ ID NO: 6 or the numerical position of the nucleotide residue corresponding to the nucleotide residue of SEQ ID NO: 6. For example, a sequence alignment algorithm as described in the present disclosure may determine the position of an amino acid or the position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of EMBOSS package (EMBOSS: The European Molecular Biology Open Software Suite, Rice et al., 2000), Trends Genet. 16: 276-277), and the like may be used, but are not limited thereto, and a sequence alignment program, a pairwise sequence comparison algorithm, etc., known in the art may be appropriately used.

As used herein, the term "weakening" of activity of a polypeptide is a concept including both cases where the activity is decreased, as compared to the endogenous activity, or the activity is absent. The weakening may be used interchangeably with terms such as inactivation, deficiency, down-regulation, decrease, reduce, attenuation, etc.

The weakening may also include a case where the activity of the polypeptide itself is decreased or eliminated due to variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or by inhibition of translation into the polypeptide, as compared to that of the natural strain, a case where the polynucleotide is not expressed at all, and/or a case where the polypeptide activity is absent even when the polynucleotide is expressed. The "endogenous activity" means the activity of a specific polypeptide originally possessed by the parent strain before change of the trait or a wild-type or unmodified microorganism when the trait is changed by genetic variation due to natural or artificial factors. The endogenous activity may be used interchangeably with "activity before modification". The fact that the activity of a polypeptide is "inactivated, deficient, decreased, down-regulated, reduced, or attenuated" as compared to the endogenous activity means that the activity of a polypeptide is lowered, as compared to the activity of a specific polypeptide originally possessed by the parent strain before change of the trait or the unmodified microorganism.

Such weakening of the activity of a polypeptide may be performed by any method known in the art, but the method is not limited thereto, and the weakening may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide activity of the present disclosure may be:
1) deletion of the entirety or a part of the gene encoding the polypeptide;
2) modification of an expression regulatory region (or expression regulatory sequence) to decrease expression of the gene encoding the polypeptide;
3) modification of an amino acid sequence constituting the polypeptide to eliminate or weaken the activity of the polypeptide (e.g., deletion/substitution/addition of one or more amino acids in the amino acid sequence);
4) modification of a gene sequence encoding the polypeptide to eliminate or weaken the activity of the polypeptide (e.g., deletion/substitution/addition of one or more nucleic acid bases in a nucleic acid base sequence of the polypeptide gene to encode the polypeptide that has been modified to eliminate or weaken the activity of the polypeptide);
5) modification of a start codon of a gene transcript encoding the polypeptide or a base sequence encoding a 5'-UTR region;
6) introduction of an antisense oligonucleotide (e.g., antisense RNA) that complementarily binds to the transcript of the gene encoding the polypeptide;
7) addition of a sequence complementary to a Shine-Dalgarno sequence in front of the Shine-Dalgarno sequence of the gene encoding the polypeptide in order to form a secondary structure to which ribosome cannot be attached;
8) addition of a promoter to be transcribed in the opposite direction to the 3' end of the open reading frame (ORF) of the gene sequence encoding the polypeptide (reverse transcription engineering, RTE); or
9) a combination of two or more selected from 1) to 8), but is not particularly limited thereto.

For example,
1) the deletion of a part or the entirety of the gene encoding the polypeptide may be removal of the entire polynucleotide encoding the intrinsic polypeptide of interest in the chromosome, replacement with a polynucleotide in which some nucleotides are deleted, or replacement with a marker gene.

Further, 2) the modification of the expression regulatory region (or expression regulatory sequence) may be deletion, insertion, non-conservative or conservative substitution, or occurrence of variation in the expression regulatory region (or expression regulatory sequence) due to a combination thereof, or replacement with a sequence exhibiting weaker activity. The expression regulatory region includes a promoter, an operator sequence, a sequence encoding a ribosome binding site, and a sequence regulating the termination of transcription and translation, but is not limited thereto.

Further, 3) and 4) the modification of the amino acid sequence or polynucleotide sequence may be deletion, insertion, or non-conservative or conservative substitution of the amino acid sequence of the polypeptide or the polynucleotide sequence encoding the polypeptide or occurrence of variation in the sequence due to a combination thereof or replacement with an amino acid sequence or a polynucleotide sequence modified to exhibit weaker activity or an amino acid sequence or a polynucleotide sequence modified to be inactive so that the activity of the polypeptide is weakened, but is not limited thereto. For example, expression of the gene may be inhibited or weakened by introducing variation into the polynucleotide sequence and forming a stop codon, but is not limited thereto.

Further, 5) the modification of a start codon of a gene transcript encoding the polypeptide or a base sequence encoding a 5'-UTR region may be, for example, substitution with a base sequence encoding another start codon having a lower polypeptide expression rate, as compared to an intrinsic start codon, but is not limited thereto.

Further, 6) the introduction of an antisense oligonucleotide (e.g., antisense RNA) that complementarily binds to the transcript of the gene encoding the polypeptide, reference may be made to documents, for example, [Weintraub, H. et al., Antisense-RNA as a molecular tool for genetic analysis, Reviews - Trends in Genetics, Vol. 1(1) 1986].

Further, 7) the addition of a sequence complementary to a Shine-Dalgarno sequence in front of the Shine-Dalgarno sequence of the gene encoding the polypeptide in order to form a secondary structure to which ribosome cannot be attached may be to make mRNA translation impossible or to slow down the mRNA translation rate.

Further, 8) the addition of a promoter to be transcribed in the opposite direction to the 3' end of the open reading frame (ORF) of the gene sequence encoding the polypeptide (reverse transcription engineering, RTE) may be to weaken the activity by making an antisense nucleotide complementary to the transcript of the gene encoding the polypeptide.

The microorganism of the present disclosure may have a branched-chain amino acid producing ability. Specifically, the branched-chain amino acid of the present disclosure may be L-valine or L-isoleucine.

As used herein, the term "microorganism (or strain)" includes all wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a polypeptide, protein, or product of interest.

Specifically, the strain of the present disclosure may be a microorganism in which the branched-chain amino acid producing ability is conferred on a parent strain having no branched-chain amino acid producing ability, or a microorganism in which the weakened ATP-dependent protease of the present disclosure or the polynucleotide encoding the same (or a vector including the polynucleotide) is introduced into the microorganism having the branched-chain amino acid producing ability, instead of a naturally present ATP-dependent protease, or the naturally present ATP-dependent protease is modified to the weakened ATP-dependent protease of the present disclosure, but is not limited thereto.

In the present disclosure, the microorganism, in which the ATP-dependent Clp protease ATP-binding subunit variant is expressed, and the branched-chain amino acid is produced, may be a microorganism characterized in that the polynucleotide of the present disclosure is included and the ATP-dependent Clp protease ATP-binding subunit variant is expressed, and thus the branched-chain amino acid producing ability is increased. Specifically, in the present disclosure, the microorganism, in which the ATP-dependent Clp protease ATP-binding subunit variant is expressed, and the branched-chain amino acid is produced, or the microorganism having the ATP-dependent Clp protease ATP-binding subunit variant-expressing ability and the branched-chain amino acid producing ability may be a microorganism, in which the ATP-dependent Clp protease ATP-binding subunit activity is weakened, and some genes in the branched-chain amino acid biosynthetic pathway are enhanced or weakened, or the ATP-dependent Clp protease ATP-binding subunit activity is weakened, and some genes in the branched-chain amino acid degradation pathway are enhanced or weakened.

The microorganism of the genus *Corynebacterium* of the present disclosure may be a microorganism, additionally, in which acetolactate synthase isozyme 1 small subunit activity is enhanced, aspartokinase activity is enhanced, homoserine dehydrogenase activity is weakened, and/or L-threonine dehydratase biosynthetic IIvA activity is enhanced.

As used herein, the term "enhancement" of polypeptide activity means that the activity of a polypeptide is increased as compared to the intrinsic activity. The enhancement may be used interchangeably with terms such as activation, up-regulation, overexpression, increase, etc. Here, activation, enhancement, up-regulation, overexpression, and increase may include both exhibiting activity that was not originally possessed and exhibiting improved activity, as compared to the intrinsic activity or activity before modification. The "intrinsic activity" means activity of a specific polypeptide originally possessed by a parent strain before change of the trait or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased", as compared to the intrinsic activity, means that the activity of a polypeptide is improved, as compared to the activity and/or concentration (expression level) of a specific polypeptide originally possessed by a parent strain before change of the trait or an unmodified microorganism.

The enhancement may be achieved through the introduction of a foreign polypeptide or the enhancement of intrinsic activity and/or concentration (expression level) of the polypeptide. The activity enhancement of a polypeptide may be confirmed by an increase in the degree of activity and the expression level of the corresponding polypeptide or in the amount of a product produced from the corresponding polypeptide.

For the activity enhancement of the polypeptide, various methods well known in the art may be applied, and the method is not limited as long as the activity of the polypeptide of interest may be enhanced, as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a start codon of a gene transcript encoding the polypeptide or a base sequence encoding a 5'-UTR region;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of a polynucleotide encoding the polypeptide;
8) analysis of the tertiary structure of the polypeptide to select the exposed site and to perform modification or chemical modification of the exposed site; or
9) a combination of two or more selected from 1) to 8), but is not particularly limited thereto.

Such enhancement of the polypeptide activity may be an increase in the activity or concentration expression level of the corresponding polypeptide, based on the activity or concentration of the polypeptide expressed in a wild-type microbial strain or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation and/or chemicals, but is not limited thereto. A method of modifying a part or the entirety of the gene may include a method of using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may include a dominant selection marker, but is not limited thereto.

The microorganism of the genus *Corynebacterium* of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Another aspect of the present disclose provides a ClpC variant, in which an amino acid sequence corresponding to positions 431 to 433 is deleted, based on an amino acid sequence represented by SEQ ID NO: 5.

The ClpC variant may be an ATP-dependent Clp protease ATP-binding subunit variant. The "ClpC" or "ATP-dependent Clp protease ATP-binding subunit" is as described above.

For example, the ClpC variant of the present disclosure may include a polypeptide represented by an amino acid sequence of SEQ ID NO: 1 or an amino acid sequence having 90% or more identity thereto.

The ClpC variant of the present disclosure may have the polypeptide represented by the amino acid sequence of SEQ ID NO: 1 or the amino acid sequence having 90% or more identity thereto, as described above, or may consist of or may consist essentially of the polypeptide. Specifically, the ClpC variant of the present disclosure may include an amino acid sequence having 91%, 92%, 93%, 94%, 95%, 96%, 97%, 98%, 99%, 99.5%, 99.7% or 99.9% or more homology or identity to the amino acid sequence represented by SEQ ID NO: 1. Further, it is apparent that variants having amino acid sequences in which some sequences are deleted, modified, substituted, conservatively substituted, or added are also included in the scope of the present disclosure as long as the amino acid sequences have such homology or identity and exhibit efficacy corresponding to that of the ClpC variant of the present disclosure.

As used herein, the term "variant" refers to a polypeptide which has an amino acid sequence different from that of the variant before modification by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased, as compared to that of the polypeptide before variation. Some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent, and is not limited thereto as long as it is a term used with the meaning of variation.

Further, the variant may include deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

As used herein, the term 'homology' or 'identity' means the degree of similarity between two given amino acid sequences or base sequences and may be expressed as a percentage. The terms 'homology and identity' may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence under moderately or highly stringent conditions. It is apparent that hybridization also includes hybridization of a polynucleotide with a polynucleotide including a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al(1988) [Proc. Natl. Acad. Sci. USA 85]: 2444. Alternatively, the homology, similarity, or identity may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387(1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al.(1970), J Mol Biol. 48:443, as announced in, for example, Smith and Waterman, Adv. Appl. Math(1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358(1979); (2) a penalty of 3.0 for each gap and an additional 0.10 penalty for each symbol in each gap (or gap opening penalty of 10, gap extension penalty of 0.5); and (3) no penalty for end gaps.

For one example of the present disclosure, the variant of the present disclosure may have the ATP-dependent Clp protease ATP-binding subunit activity. Further, the variant of the present disclosure may have an activity to increase the production of branched-chain amino acids, as compared to the wild-type polypeptide having the ATP-dependent Clp protease ATP-binding subunit activity.

Still another aspect of the present disclosure provides a polynucleotide encoding the ClpC variant of the present disclosure.

The "ClpC variant" and "ATP-dependent Clp protease ATP-binding subunit variant" are as described above.

As used herein, the term "polynucleotide" is a DNA or RNA strand having a certain length or more as a polymer of nucleotides in which nucleotide monomers are connected in a long chain by covalent bonds, and more specifically, it means a polynucleotide fragment encoding the variant.

The polynucleotide encoding the ClpC variant of the present disclosure may include a base sequence encoding a polypeptide represented by the amino acid sequence of SEQ ID NO: 1 or an amino acid sequence having 90% or more identity thereto. As an example of the present disclosure, the polynucleotide of the present disclosure may have or include a polynucleotide represented by a nucleotide sequence of SEQ ID NO: 2 or a nucleotide sequence having 90% or more identity thereto. Further, the polynucleotide of the present disclosure may consist of or may consist essentially of the polynucleotide represented by the nucleotide sequence of SEQ ID NO: 2.

In the polynucleotide of the present disclosure, various modifications may be made in the coding region as long as the amino acid sequence of the variant of the present disclosure is not changed, in consideration of codon degeneracy or codons preferred in organisms that are intended to express the variant of the present disclosure.

Further, the polynucleotide of the present disclosure may include a probe that may be prepared from a known gene sequence, for example, a sequence without limitation as long as it is a sequence that may hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). Examples thereof include conditions in which polynucleotides having higher homology or identity, namely, polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or washing conditions for common Southern hybridization, in which washing is performed once, specifically, two to three times at a salt concentration and temperature equivalent to 60°C, 1X SSC, 0.1% SDS, specifically 60°C, 0.1X SSC, 0.1% SDS, more specifically, 68°C, 0.1X SSC, 0.1% SDS.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Therefore, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using hybridization conditions including a hybridization step at a Tm value of 55°C and the above-described conditions. The Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector including the polynucleotide of the present disclosure.

The vector may be an expression vector for expressing the polynucleotide in host cells, but is not limited thereto.

The vector of the present disclosure refers to a DNA construct including a polynucleotide sequence of interest operably linked to a suitable regulatory sequence so that the gene of interest may be introduced into a suitable host. The regulatory sequence may include a promoter capable of initiating transcription, any operator sequence for regulating the transcription, a sequence encoding a suitable mRNA ribosome binding site, and a sequence regulating termination of transcription and translation. The vector may be transformed into a suitable host cell and then replicated or function independently of the host genome, or may be integrated into the genome itself. For example, a polynucleotide of interest in the chromosome may be replaced with a modified polynucleotide through a vector for intracellular chromosome insertion. Insertion of the polynucleotide into the chromosome may be performed by any method known in the art, for example, homologous recombination, but is not limited thereto.

The vector used in the present disclosure is not particularly limited, but any vector known in the art may be used. Examples of commonly used vectors may include natural or recombinant plasmids, cosmids, viruses, and bacteriophages. For example, pWE15, M13, MBL3, MBL4, IXII, ASHII, APII, t10, t11, Charon4A, Charon21A, or the like may be used as a phage vector or a cosmid vector. pDZ system, pBR system, pUC system, pBluescript II system, pGEM system, pTZ system, pCL system, pET system, or the like may be used as a plasmid vector. Specifically, pDZ, pDC, pDCM2, pACYC177, pACYC184, pCL, pECCG117, pUC19, pBR322, pMW118, pCC1 BAC vectors or the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for identifying the chromosome insertion. The selection marker is for selecting the cells transformed with vectors, i.e., for identifying the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target protein is introduced into a host cell so that the protein encoded by the polynucleotide may be expressed in the host cell. The transformed polynucleotide may be located by being inserted into the chromosome of the host cell or located outside the chromosome as long as it may be expressed in the host cell. Further, the polynucleotide includes DNA and RNA encoding a protein of interest. The polynucleotide may be introduced in any form as long as it may be introduced into a host cell and then expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

The method of transforming the vector of the present disclosure includes any method of introducing a nucleic acid into a cell, and may be performed by selecting an appropriate standard technique, as known in the art, according to the host cell. For example, the method may include electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method, etc., but is not limited thereto.

Further, as used herein, the term "operably linked" means that the polynucleotide sequence is functionally linked to a promoter sequence that initiates and mediates transcription of the polynucleotide encoding the protein of interest of the present disclosure.

Still another aspect of the present disclosure provides a strain of the genus *Corynebacterium,* the strain including any one or more of the variant of the present disclosure, the polynucleotide of the present disclosure, or the vector of the present disclosure.

The strain of the present disclosure may be a strain including any one or more of the variant of the present disclosure, the polynucleotide of the present disclosure, and the vector including the polynucleotide of the present disclosure; a strain modified to express the variant of the present disclosure or the polynucleotide of the present disclosure; a strain (e.g., recombinant strain) expressing the variant of the present disclosure or the polynucleotide of the present disclosure; or a strain (e.g., recombinant strain) having the variant activity of the present disclosure, but is not limited thereto.

For example, the strain of the present disclosure is a cell or microorganism that is transformed with a vector including the polynucleotide of the present disclosure or a polynucleotide encoding the ClpC variant of the present disclosure and expresses the ClpC variant of the present disclosure. With respect to the objects of the present disclosure, the strain of the present disclosure may include all microorganisms that are able to produce branched-chain amino acids by including the ClpC variant of the present disclosure. For example, the strain of the present disclosure may be a recombinant strain in which the polynucleotide encoding the ClpC variant of the present disclosure is introduced into a microorganism producing branched-chain amino acids to express the ClpC variant, and thus the branched-chain amino acid producing ability is enhanced. The recombinant strain having the enhanced branched-chain amino acid producing ability may be a microorganism having enhanced branched-chain amino acid producing ability, as compared to a natural wild-type microorganism or a ClpC unmodified microorganism (i.e., a microorganism expressing a wild-type aldehyde dehydrogenase (SEQ ID NO: 5) or a microorganism that does not express the modified (SEQ ID NO: 1) protein), but is not limited thereto. For example, the ClpC unmodified microorganism which is a target strain for comparison of the increase in the branched-chain amino acid producing ability may be CA08-0072 strain (KCCM11201P, US 8465962 B2) or KCJI-38 (KCCM11248P, Korean Patent NO. 10-1335789), but is not limited thereto.

As used herein, the term "unmodified microorganism" does not exclude strains including mutations that may occur naturally in microorganisms, and may be a wild-type strain or a natural strain itself or may be a strain before the trait is changed by genetic variation due to natural or artificial factors. For example, the unmodified microorganism may be a strain into which the ClpC variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

Still another aspect of the present disclosure provides a method of producing branched-chain amino acids, the method including the step of culturing, in a medium, the microorganism of the genus *Corynebacterium,* in which the ATP-dependent protease activity of the present disclosure is weakened, as compared to the unmodified microorganism, or a microorganism of the genus *Corynebacterium* including the ClpC variant of the present disclosure, the polynucleotide encoding the ClpC variant of the present disclosure, or the vector of the present disclosure.

As used herein, the term "culture" means growing the microorganism of the genus *Corynebacterium* of the present disclosure under appropriately controlled environmental conditions. The culture process of the present disclosure may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

As used herein, the term "medium" means a mixed substance containing nutrients required to culture the microorganism of the genus *Corynebacterium* of the present disclosure as a main component, and the medium supplies nutrients and growth factors, including water, which are indispensable for survival and development. Specifically, as the medium and other culture conditions used for culture of the microorganism of the genus *Corynebacterium* of the present disclosure, any one may be used without particular limitation as long as it is a medium used for common culture of microorganisms. The microorganism of the genus *Corynebacterium* of the present disclosure may be cultured in a common medium containing proper carbon sources, nitrogen sources, phosphorus sources, inorganic compounds, amino acids and/or vitamins, etc., while controlling the temperature, pH, etc. under aerobic conditions.

Specifically, the culture medium for the strain of the genus *Corynebacterium* may be found in the document ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, maltose, etc.; sugar alcohols such as mannitol, sorbitol, etc., organic acids such as pyruvic acid, lactic acid, citric acid, etc.; amino acids such as glutamic acid, methionine, lysine, etc.; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (i.e., molasses converted to reducing sugar) may be used, and appropriate amounts of other carbon sources may be used in various manners without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, etc.; and organic nitrogen sources such as amino acids such as glutamic acid, methionine, glutamine, etc., peptone, NZ-amine, meat extract, yeast extract, malt extract, corn steep liquor, casein hydrolysate, fish or decomposition products thereof, and skim soybean cake or decomposition products thereof, etc. may be used. These nitrogen sources may be used alone or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, etc. may be used. In addition to these compounds, amino acids, vitamins and/or suitable precursors, etc. may be included. These components or precursors may be added to the medium batchwise or continuously, but is not limited thereto.

Further, during the culture of the microorganism of the genus *Corynebacterium* of the present disclosure, pH of the medium may be adjusted by adding compounds such as ammonium hydroxide, potassium hydroxide, ammonia, phosphoric acid, or sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but is not limited thereto.

In the culture of the present disclosure, the culture temperature may be maintained at 20°C to 45°C, specifically, at 25°C to 40°C, and the strain may be cultured for about 10 hours to about 160 hours, but are not limited thereto.

The branched-chain amino acids produced through the culture of the present disclosure may be secreted into the medium or may remain in the cells.

The method of producing branched-chain amino acids of the present disclosure may further include the step of preparing the microorganism of the genus *Corynebacterium* of the present disclosure, the step of preparing a medium for culture of the microorganism, or a combination of these steps (in any order), for example, prior to the culture step.

The method of producing branched-chain amino acids of the present disclosure may further include the step of recovering branched-chain amino acids from the medium according to the culture (the medium subjected to the culture) or from the microorganism of the genus *Corynebacterium* of the present disclosure. The recovery step may be further included after the culture step.

The recovery may be to collect branched-chain amino acids of interest by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular sieve chromatography (gel filtration), adsorption chromatography, ion-exchange chromatography, and affinity chromatography, HPLC, or a combination thereof may be used. The branched-chain amino acids of interest may be recovered from the medium or microorganism by way of a suitable method known in the art.

Further, the method of producing branched-chain amino acids of the present disclosure may further include a purification step. The purification may be performed by way of a suitable method known in the art. For example, when the method of producing branched-chain amino acids of the present disclosure includes both the recovery step and the purification step, the recovery step and the purification step may be performed discontinuously (or continuously) regardless of the order, or may be performed simultaneously or by being combined into one step, but is not limited thereto.

In the method of the present disclosure, the variant, polynucleotide, vector, microorganism and branched-chain amino acid, and the like are as described in other aspects.

Still another aspect of the present disclosure provides a composition for producing branched-chain amino acids, the composition including the variant of the present disclosure, a polynucleotide encoding the variant, a vector including the polynucleotide, or the microorganism of the present disclosure; a medium in which the microorganism of the present disclosure has been cultured; or a combination of two or more thereof.

The composition of the present disclosure may further include arbitrary suitable excipients to be commonly used in compositions for producing branched-chain amino acids. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, branched-chain amino acids, and the like are as described in other aspects.

Still another aspect of the present disclosure provides use of the microorganism of the genus *Corynebacterium,* in which ATP-dependent protease activity is weakened, as compared to an unmodified microorganism, in the production of branched-chain amino acids.

Still another aspect of the present disclosure provides use of the ClpC variant, in which an amino acid sequence corresponding to positions 431 to 433 is deleted, based on the amino acid sequence represented by SEQ ID NO: 5, or the polynucleotide encoding the ClpC variant, in the production of branched-chain amino acids.

Hereinafter, the present disclosure will be described in more detail with reference to exemplary embodiments. However, the following exemplary embodiments are only preferred embodiments for illustrating the present disclosure, and thus are not intended to limit the scope of the present disclosure thereto. Meanwhile, technical matters not described in the present specification can be sufficiently understood and easily implemented by those skilled in the technical field of the present disclosure or similar technical fields.

### Example 1. Selection of variants with enhanced L-valine producing ability through artificial mutation method

### 1-1. Artificial mutagenesis through UV irradiation

In order to select a variant strain having enhanced L-valine producing ability, *Corynebacterium glutamicum* CA08-0072 (KCCM11201P, US 8465962 B2) which is an L-valine-producing NTG strain was spread on a nutrient medium containing agar, and cultured at 30°C for 36 hours.

Hundreds of colonies obtained therethrough were irradiated with UV at room temperature to induce random mutations on the genome of the strain.

The composition of the nutrient medium is as follows.

### <Nutrient medium (pH 7.2)>

10 g of glucose, 5 g of beef extract, 10 g of polypeptone, 2.5 g of sodium chloride, 5 g of yeast extract, 20 g of agar, 2 g of urea (based on 1 liter of distilled water)

### 1-2. Fermentation titer test of mutagenesis strain and Selection of strain

In order to select variant strains with enhanced L-valine producing ability, as compared to *Corynebacterium glutamicum* CA08-0072 which was used as the parent strain in Example 1-1, a fermentation titer test was conducted on random mutation-induced strains.

In detail, each colony was sub-cultured in a nutrient medium, and each strain was seeded into a 250 ml corner-baffle flask containing 25 ml of a production medium, and cultured at 30°C for 72 hours under shaking at 200 rpm. The compositions of the nutrient medium and the production medium are as follows, respectively.

### <Nutrient medium (pH 7.2)>

10 g of glucose, 5 g of beef extract, 10 g of polypeptone, 2.5 g of sodium chloride, 5 g of yeast extract, 20 g of agar, 2 g of urea (based on 1 liter of distilled water)

### <Production medium (pH 7.0)>

100 g of glucose, 40 g of ammonium sulfate, 2.5 g of soy protein, 5 g of corn steep solids, 3 g of urea, 1 g of dipotassium phosphate, 0.5 g of magnesium sulfate heptahydrate, 100 µg of biotin, 1 mg of thiamine-HCl, 2 mg of calcium pantothenate, 3 mg of nicotinamide, 30 g of calcium carbonate (based on 1 liter of distilled water).

Thereafter, the concentrations of L-valine were analyzed using HPLC, and the analyzed concentrations of L-valine are shown in Table 1 below.

**[Table 1]**

| | Strain name | L-Valine (g/L) |
|---|---|---|
| Control group | CA08-0072 | 2.7 |
| Experimental group | M1 | 3.0 |
| | M2 | 2.8 |
| | M3 | 2.5 |
| | M4 | 4.8 |
| | M5 | 3.5 |
| | M6 | 3.3 |
| | M7 | 2.9 |
| | M8 | 3.9 |
| | M9 | 3.5 |
| | M10 | 2.1 |
| | M11 | 1.1 |
| | M12 | 2.9 |
| | M13 | 2.5 |
| | M14 | 3.1 |
| | M15 | 4.7 |
| | M16 | 3.2 |

Referring to Table 1, the M4 strain was selected, which shows the highest increase in the L-valine production as compared to the control CA08-0072 strain.

### Example 2. Identification of variation through gene sequencing

Major genes of the M4 strain with enhanced L-valine producing ability in Example 1 were sequenced, and compared with those of the CA08-0072 strain and the wild-type *Corynebacterium glutamicum* strain ATCC14067. As a result, it was confirmed that the M4 strain includes a variation at a specific position of the open reading frame (ORF) of clpC, which is one of the hexameric ATPase-active chaperon subunits constituting the ATP-dependent protease. Specifically, it was confirmed that the M4 strain had a deletion of nucleotides at positions 1,291 to 1,299 (SEQ ID NO: 4) in the sequence represented by SEQ ID NO: 6. The sequence represented by SEQ ID NO: 6 is a sequence commonly included in the ORF of clpC of the wild-type *Corynebacterium glutamicum* (ATCC14067, ATCC13032, and ATCC13869).

In the following Examples, it was attempted to confirm whether the variation affects the production of amino acids by the microorganisms of the genus *Corynebacterium.*

### Example 3. Production of variation-introduced strain and Examination of L-valine producing ability

### 3-1. Production of strain by introducing variation into Corynebacterium glutamicum CA08-0072 and Evaluation of L-valine producing ability

A vector for introducing a nucleotide sequence (SEQ ID NO: 2), in which nucleotides at positions 1,291 to 1,299 (SEQ ID NO: 4) in a sequence represented by SEQ ID NO: 6 were deleted, into the L-valine-producing NTG strain *Corynebacterium glutamicum* CA08-0072, was constructed. In detail, genomic DNA of the wild-type *Corynebacterium glutamicum* strain ATCC14067 was extracted using a G-Spin Total DNA Extraction Mini Kit (Intron, Cat. No 17045) according to a protocol provided in the kit. PCR was performed using each genomic DNA as a template, and primers of SEQ ID NOS: 7 and 8 and primers of SEQ ID NOS: 9 and 10 to obtain DNA fragments (A and B), respectively. Primer sequences used here are shown in Table 2 below.

**[Table 2]**

| SEQ ID NO. | Sequence name | Sequence |
|---|---|---|
| 7 | P1 | ctat tctaga tccagagaccctcaaggacaagcag |
| 8 | P2 | ggacggtgcggtcatgcgcatgcgggcgcc |
| 9 | P3 | ggcgcccgcatgcgcatgaccgcaccgtcc |
| 10 | P4 | ctat tctaga tcgatttggatgagggaatcatcg |

Overlapping PCR was performed using the two fragments as templates and primers of SEQ ID NOS: 7 and 10 to obtain a PCR product of 1,040 bp (hereinafter, referred to as "variation-introduced fragment"). PCR was performed as follows: denaturation at 94°C for 5 minutes; 25 cycles of denaturation at 94°C for 30 seconds, annealing at 55°C for 30 seconds, and polymerization at 72°C for 60 seconds; and polymerization at 72°C for 7 minutes. The variation-introduced fragments were treated with a restriction enzyme Xbal (New England Biolabs, Beverly, MA), and then ligated with a pDZ vector (U.S. Patent NO. 9109242 and International Patent Publication No. 2008-033001) unable to replicate in *Corynebacterium glutamicum,* which had been treated with the same restriction enzyme, using T4 ligase (New England Biolabs, Beverly, MA). The prepared gene was transformed into *E*. *coli* DH5α, and selected in LB medium containing 25 mg/L kanamycin, and DNA was obtained using a DNA-spin plasmid DNA purification kit (Intron) to construct a recombinant plasmid pDZ-clpC_M4.

The recombinant plasmid pDZ-clpC_M4 prepared above was transformed into the L-valine-producing strain, *Corynebacterium glutamicum* CA08-0072 by homologous recombination on the chromosome (van der Rest et al., Appl Microbiol Biotechnol 52:541-545, 1999). The strain in which the vector was inserted onto the chromosome by homologous recombination was selected in a medium containing 25 mg/L kanamycin. Then, PCR was performed on the *Corynebacterium glutamicum* transformant, in which the secondary cross-over was completed, using primers of SEQ ID NOS: 7 and 10 to prepare a strain, in which the variation was introduced into the ORF of clpC on the chromosome. The recombinant strain was named *Corynebacterium glutamicum* CA08-0072-clpC_M4.

To compare the L-valine producing ability between the prepared L-valine-producing strain *Corynebacterium glutamicum* CA08-0072, and CA08-0072-clpC_M4, a fermentation titer test was performed.

In detail, each strain was sub-cultured in a nutrient medium, and seeded into a 250 ml corner-baffle flask containing 25 ml of a production medium, and cultured at 30°C for 72 hours under shaking at 200 rpm. The compositions of the nutrient medium and the production medium are as follows, respectively.

### <Nutrient medium (pH 7.2)>

10 g of glucose, 5 g of beef extract, 10 g of polypeptone, 2.5 g of sodium chloride, 5 g of yeast extract, 20 g of agar, 2 g of urea (based on 1 liter of distilled water)

### <Production medium (pH 7.0)>

100 g of glucose, 40 g of ammonium sulfate, 2.5 g of soy protein, 5 g of corn steep solids, 3 g of urea, 1 g of dipotassium phosphate, 0.5 g of magnesium sulfate heptahydrate, 100 µg of biotin, 1 mg of thiamine-HCl, 2 mg of calcium pantothenate, 3 mg of nicotinamide, 30 g of calcium carbonate (based on 1 liter of distilled water).

Thereafter, the concentrations of L-valine were analyzed using HPLC, and the analyzed concentrations of L-valine are shown in Table 3 below.

**[Table 3]**

| | Strain | L-Valine (g/L) | | | |
|---|---|---|---|---|---|
| | | Batch 1 | Batch 2 | Batch 3 | Mean |
| Control group | CA08-0072 | 2.8 | 2.6 | 2.7 | 2.7 |
| Experimental group | CA08-0072-clpC_M4 | 3.2 | 3.1 | 3.0 | 3.1 |

As shown in the results of Table 3, it was confirmed that the L-valine producing ability of the CA08-0072-clpC_M4 strain increased by 14%, as compared to the control group. As a result, it was confirmed that the L-valine producing ability may be improved through ORF variation of clpC.

The CA08-0072-clpC_M4 was named CA08-1542, and deposited at the Korean Culture Center of Microorganisms under the Budapest Treaty on July 2, 2020, with Accession No. KCCM12755P.

### 3-2. Production of strain by introducing variation into Corynebacterium glutamicum CJ7V and Evaluation of L-valine producing ability

In order to examine whether the same effect is also observed in other L-valine-producing strains belonging to the *Corynebacterium glutamicum,* a type of variation [ilvN(A42V); Biotechnology and Bioprocess Engineering, June 2014, Volume 19, Issue 3, pp 456-467] was introduced into the wild-type strain *Corynebacterium glutamicum* ATCC14067 to produce a strain with improved L-valine producing ability.

In detail, genomic DNA of the wild-type *Corynebacterium glutamicum* strain ATCC14067 was extracted using a G-Spin Total DNA Extraction Mini Kit. To construct a vector for introducing A42V variation into ilvN gene, PCR was performed using the genomic DNA as a template, and primers of SEQ ID NOS: 11 and 12 and primers of SEQ ID NOS: 13 and 14 to obtain DNA fragments (A and B), respectively. Primer sequences used here are shown in Table 4 below.

**[Table 4]**

| SEQ ID NO. | Sequence name | Sequence |
|---|---|---|
| 11 | P5 | aatttctagaggcagaccctattctatgaagg |
| 12 | P6 | agtgtttcggtctttacagacacgagggac |
| 13 | P7 | gtccctcgtgtctgtaaagaccgaaacact |
| 14 | P8 | aatttctagacgtgggagtgtcactcgcttgg |

Overlapping PCR was performed using the two fragments as templates and primers of SEQ ID NOS: 11 and 14 to obtain a PCR product of 1044 bp (hereinafter, referred to as "variation-introduced fragment"). PCR was performed as follows: denaturation at 94°C for 5 minutes; 25 cycles of denaturation at 94°C for 30 seconds, annealing at 55°C for 30 seconds, and polymerization at 72°C for 60 seconds; and polymerization at 72°C for 7 minutes. As a result, polynucleotides of 537 bp were obtained for both the fragments A and B. The variation-introduced fragments thus obtained were treated with a restriction enzyme Xbal, and then ligated with a pDZ vector treated with the same restriction enzyme using T4 ligase. The prepared gene was transformed into E. *coli* DH5α, and selected in LB medium containing 25 mg/L kanamycin, and DNA was obtained using a DNA-spin plasmid DNA purification kit. The vector for the purpose of introducing the A42V variation into ilvN gene was named pDZ-ilvN(A42V).

Thereafter, the recombinant plasmid pDZ-ilvN(A42V) prepared above was transformed into the wild-type *Corynebacterium glutamicum* ATCC14067 by homologous recombination on the chromosome. The strain in which the vector was inserted onto the chromosome by homologous recombination was selected in a medium containing 25 mg/L kanamycin. Then, PCR was performed on the *Corynebacterium glutamicum* transformant, in which the secondary cross-over had been completed, using primers of SEQ ID NOS: 11 and 14 to amplify the gene fragment, and then the variation-introduced strain was identified through gene sequencing analysis. The recombinant strain, in which the variation was introduced, was named *Corynebacterium glutamicum* CJ7V.

The *Corynebacterium glutamicum* CJ7V was transformed with pDZ-clpC_M4 in the same manner as in Example 3-1 to prepare a strain in which the variation was introduced into ORF of clpC, and the strain was named CJ7V-clpC_M4.

To compare the L-valine producing ability of the prepared strain, the strain was cultured in the same manner as in Example 3-1, and the concentration of L-valine was analyzed, and the analyzed concentration of L-valine is shown in Table 5 below.

**[Table 5]**

| | Strain | L-Valine (g/L) | | | |
|---|---|---|---|---|---|
| | | Batch 1 | Batch 2 | Batch 3 | Mean |
| Control group | CJ7V | 2.1 | 2.2 | 2.2 | 2.2 |
| Experimental group | CJ7V-clpC_M4 | 2.6 | 2.4 | 2.5 | 2.5 |

As shown in the results of Table 5, it was confirmed that the L-valine producing ability of the CJ7V-clpC_M4 strain increased by 14%, as compared to the control group. In other words, it was also confirmed that the L-valine producing ability may be improved through ORF variation of clpC gene in the microorganism of the genus *Corynebacterium.*

### 3-3. Production of strain by introducing variation into Corynebacterium glutamicum CJ8V and Evaluation of L-valine producing ability

In order to examine whether the same effect is also observed in other L-valine-producing strains belonging to the *Corynebacterium glutamicum,* a type of variation [ilvN(A42V)] was introduced into the wild-type strain *Corynebacterium glutamicum* ATCC13869 in the same manner as in Example 3-2 to produce a variant strain with L-valine producing ability. The recombinant strain was named *Corynebacterium glutamicum* CJ8V.

To prepare a strain, in which clpC variation was introduced into the *Corynebacterium glutamicum* CJ8V, the recombinant vector pDZ-clpC_M4 prepared in Example 3-1 was transformed into CJ8V. The strain in which the vector was inserted onto the chromosome by homologous recombination was selected in a medium containing 25 mg/L kanamycin. Then, the *Corynebacterium glutamicum* transformant, in which the secondary cross-over was completed, was examined using primers of SEQ ID NOS: 7 and 10, and the clpC variation-introduced strain was identified. The recombinant strain, as identified above, was named *Corynebacterium glutamicum* CJ8V-clpC_M4.

To compare the L-valine producing ability of the prepared strain, the strain was cultured in the same manner as in Example 3-1, and the concentration of L-valine was analyzed, and the analyzed concentration of L-valine is shown in Table 6 below.

**[Table 6]**

| | Strain | L-Valine (g/L) | | | |
|---|---|---|---|---|---|
| | | Batch 1 | Batch 2 | Batch 3 | Mean |
| Control group | CJ8V | 1.8 | 1.9 | 1.9 | 1.9 |
| Experimental group | CJ8V-clpC_M4-14067 | 2.2 | 2.1 | 2.1 | 2.13 |

As shown in the results of Table 6, it was confirmed that the L-valine producing ability of the CJ8V-clpC_M4 strain increased by 12%, as compared to the control group. In other words, it was also confirmed that the L-valine producing ability may be improved through ORF variation of clpC gene in the microorganism of the genus *Corynebacterium.*

### Example 4. Production of isoleucine-producing strain and Evaluation of production ability

### 4-1. Production of strain by introducing clpC ORF variation into L-isoleucine producing Corynebacterium glutamicum KCCM11248P strain and Evaluation thereof

A strain was prepared, in which the recombinant plasmid pDZ-clpC_M4 prepared in Example 3-1 was introduced into an L-isoleucine producing strain *Corynebacterium glutamicum* KCJI-38 (KCCM11248P, U.S. Patent No. 9885093) in the same manner as in Example 3 by homologous recombination on the chromosome, and the strain was named KCJI-38-clpC_M4. The prepared strain was cultured by the following method, and its isoleucine producing ability was compared.

In detail, each strain was seeded into a 250 ml corner-baffle flask containing 25 ml of a seed medium, and cultured at 30°C for 20 hours under shaking at 200 rpm. Thereafter, 1 ml of the seed culture was seeded into a 250 ml corner-baffle flask containing 24 ml of a production medium, and cultured at 30°C for 48 hours under shaking at 200 rpm. The compositions of the seed medium and the production medium are as follows, respectively.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄ 7H₂O, 100 µg of biotin, 1000 µg of thiamine-HCl, 2000 µg of calcium pantothenate, 2000 µg of nicotinamide (based on 1 liter of distilled water)

### <Production medium (pH 7.0)>

50 g of glucose, 12.5 g of (NH₄)₂SO₄, 2.5 g of soy protein, 5 g of corn steep solids, 3 g of urea, 1 g of KH₂PO₄, 0.5 g of MgSO₄7H₂O, 100 µg of biotin, 1000 µg of thiamine-HCl, 2000 µg of calcium pantothenate, 3000 µg of nicotinamide, 30 g of CaCOs (based on 1 liter of distilled water).

After the culture was completed, the L-isoleucine producing ability was measured using HPLC. The concentrations of L-isoleucine in the culture of each experimented strain are shown in Table 7 below.

**[Table 7]**

| | Strain | L-isoleucine (g/L) | | | |
|---|---|---|---|---|---|
| | | Batch 1 | Batch 2 | Batch 3 | Mean |
| Control group | KCJI-38 | 1.4 | 1.5 | 1.3 | 1.4 |
| Experimental group | KCJI-38-clpC_M4 | 2.1 | 2.4 | 2.0 | 2.17 |

As shown in Table 7, it was confirmed that the concentration of L-isoleucine increased by about 55% in KCJI-38-clpC_M4 into which the clpC variation was introduced, as compared to the L-isoleucine producing strain KCJI-38. This confirmed that the L-isoleucine producing ability may be improved through the variation of the clpC gene. The above results indicate that the introduction of the clpC ORF variation into the L-isoleucine producing strain of the genus *Corynebacterium* is effective in the production of L-isoleucine.

The KCJI-38-clpC_M4 was named CA10-3123, and deposited at the Korean Culture Center of Microorganisms under the Budapest Treaty on December 1, 2020, with Accession No. KCCM12858P.

### 4-2. Production of L-isoleucine strain by introducing clpC ORF variation into wild-type Corynebacterium glutamicum strain ATCC13032 and Evaluation of L-isoleucine producing ability

To examine the effect of the introduction of the clpC-M4 variation on the L-isoleucine producing ability, strains were prepared by introducing, into *Corynebacterium glutamicum* ATCC13032 (hereinafter, referred to as WT) strain, a known aspartokinase variant (lysC(L377K)) (US 10662450 B2), homoserine dehydrogenase variant (hom(R407H)) (Appl. Microbiol. Biotechnol. 45, 612-620 (1996)), or L-threonine dehydratase variant (ilvA(V323A)) (Appl. Enviro. Microbiol., Dec. 1996, p.4345-4351), and their L-isoleucine producing abilities were compared.

In detail, PCR was performed using the chromosome of WT as a template and primers of SEQ ID NOS: 15 and 16 or SEQ ID NOS: 17 and 18. Primer sequences used here are shown in Table 8 below.

**[Table 8]**

| SEQ ID NO. | Sequence name | Nucleotide sequence |
|---|---|---|
| 15 | P9 | tcctctaqaGCTGCGCAGTGTTGAATACG |
| 16 | P10 | TGGAAATCttTTCGATGTTCACGTTGACAT |
| 17 | P11 | ACATCGAAaaGATTTCCACCTCTGAGATTC |
| 18 | P12 | gactctagaGTTCACCTCAGAGACGATTA |

PCR was performed as follows: denaturation at 95°C for 5 minutes; 30 cycles of denaturation at 95°C for 30 seconds, annealing at 55°C for 30 seconds, and polymerization at 72°C for 30 seconds; and polymerization at 72°C for 7 minutes. As a result, a DNA fragment of 509 bp at the 5' upstream and a DNA fragment of 520 bp at the 3' downstream, centering on the variation of the lysC gene, were obtained, respectively. PCR was performed using the amplified two DNA fragments as templates and primers of SEQ ID NOS: 15 and 18. PCR was performed as follows: denaturation at 95°C for 5 minutes; 30 cycles of denaturation at 95°C for 30 seconds, annealing at 55°C for 30 seconds, and polymerization at 72°C for 30 seconds; and polymerization at 72°C for 7 minutes. As a result, a DNA fragment of 1011 bp was amplified, the DNA fragment including the lysC gene variation (L377K) encoding the aspartokinase variant in which leucine at position 377 was substituted with lysine. The pDZ vector and the amplified DNA fragment of 1011 bp were treated with a restriction enzyme Xbal, ligated using DNA ligase, and then cloned to obtain a plasmid, which was named pDZ-lysC (L377K).

The pDZ-lysC (L377K) vector obtained above was introduced into the WT strain by an electric pulse method (Appl. Microbiol. Biothcenol.(1999, 52:541-545)) and then a transformed strain was obtained in a selective medium containing 25 mg/L kanamycin. A strain WT:: lysC (L377K) was obtained, in which the nucleotide variation was introduced into the lysC gene by the DNA fragment inserted into the chromosome through the secondary recombination process.

In addition, in order to prepare a vector for introducing horn (R407H), PCR was performed using WT genomic DNA as a template and primers of SEQ ID NOS: 19 and 20 and SEQ ID NOS: 21 and 22. Primer sequences used here are shown in Table 9 below.

**[Table 9]**

| SEQ ID NO. | Sequence name | Sequence |
|---|---|---|
| 19 | P13 | tcctctagaCTGGTCGCCTGATGTTCTAC |
| 20 | P14 | CACGATCAGATGTGCATCATCAT |
| 21 | P15 | ATGATGATGCACATCTGATCGTG |
| 22 | P16 | gactctagaTTAGTCCCTTTCGAGGCGGA |

PCR was performed as follows: denaturation at 95°C for 5 minutes; 30 cycles of denaturation at 95°C for 30 seconds, annealing at 55°C for 30 seconds, and polymerization at 72°C for 30 seconds; and polymerization at 72°C for 7 minutes. As a result, a DNA fragment of 220 bp at the 5' upstream and a DNA fragment of 220 bp at the 3' downstream, centering on the variation of the horn gene, were obtained, respectively. PCR was performed using the amplified two DNA fragments as templates and primers of SEQ ID NOS: 19 and 20. PCR was performed as follows: denaturation at 95°C for 5 minutes; 30 cycles of denaturation at 95°C for 30 seconds, annealing at 55°C for 30 seconds, and polymerization at 72°C for 30 seconds; and polymerization at 72°C for 7 minutes. As a result, a DNA fragment of 440 bp was amplified, the DNA fragment including the horn gene variation. The pDZ vector used above and the amplified DNA fragment of 440 bp were treated with a restriction enzyme Xbal, ligated using DNA ligase, and then cloned to obtain a plasmid, which was named pDZ-hom(R407H).

The pDZ-hom(R407H) vector obtained above was introduced into the WT::lysC(L377K) strain by an electric pulse method and then transformed strain was obtained in a selective medium containing 25 mg/L kanamycin. A strain WT::lysC(L377K)-hom(R407H) was obtained, in which the nucleotide variation was introduced into the horn gene by the DNA fragment inserted into the chromosome through the secondary recombination process.

In the same manner as the above Example, a strain was prepared by introducing the recombinant plasmid pDZ-clpC_M4 prepared in Example 3-1 into the WT::lysC(L377K)-hom(R407H) strain by homologous recombination on the chromosome, and named WT::lysC(L377K)-hom(R407H)-clpC_M4.

Meanwhile, to prepare a vector, in which a known ilvA(V323A) variation (S. Morbach et al., Appl. Enviro. Microbiol., 62(12): 4345-4351, 1996) was introduced into the ilvA gene, a pair of primers (SEQ ID NOS: 23 and 24) for amplifying the 5' upstream and a pair of primers (SEQ ID NOS: 25 and 26) for amplifying the 3' downstream, centering on the position of the variation, were designed, respectively. In the primers of SEQ ID NOS: 23 and 26, a BamHI restriction enzyme site (underlined) was inserted at each end. In the primers of SEQ ID NOS: 24 and 25, the variation of nucleotide substitution (underlined) was placed at the site which was designed to cross each other. Primer sequences designed here are shown in Table 10 below.

**[Table 10]**

| SEQ ID NO. | Sequence name | Sequence |
|---|---|---|
| 23 | P17 | ACGGATCCCAGACTCCAAAGCAAAAGCG |
| 24 | P18 | ACACCACGgCAGAACCAGGTGCAAAGGACA |
| 25 | P19 | CTGGTTCTGcCGTGGTGTGCATCATCTCTG |
| 26 | P20 | ACGGATCCAACCAAACTTGCTCACACTC |

PCR was performed using the chromosome of WT as a template and primers of SEQ ID NO: 23 and SEQ ID NO: 24, SEQ ID NO: 25 and SEQ ID NO: 26. PCR was performed as follows: denaturation at 95°C for 5 minutes; 30 cycles of denaturation at 95°C for 30 seconds, annealing at 55°C for 30 seconds, and polymerization at 72°C for 30 seconds; and polymerization at 72°C for 7 minutes. As a result, a DNA fragment of 627 bp at the 5' upstream and a DNA fragment of 608 bp at the 3' downstream, centering on the variation of the ilvA gene, were obtained, respectively. PCR was performed using the amplified two DNA fragments as templates and primers of SEQ ID NOS: 23 and 26. PCR was performed as follows: denaturation at 95°C for 5 minutes; 30 cycles of denaturation at 95°C for 30 seconds, annealing at 55°C for 30 seconds, and polymerization at 72°C for 60 seconds; and polymerization at 72°C for 7 minutes. As a result, a DNA fragment of 1217 bp was amplified, the DNA fragment including the variation of ilvA gene encoding the IlvA variant in which L-valine at position 323 was substituted with alanine. The pECCG117 (Korea Patent No. 10-0057684) vector and the DNA fragment of 1217 bp were treated with a restriction enzyme BamHI, ligated using DNA ligase, and cloned to obtain a plasmid, which was named pECCG117-ilvA (V323A).

An ATCC13032::hom(R407H)-lysC(L377K)-clpC/pECCG117-ilvA(V323A) strain was prepared by introducing the pECCG117-ilvA(V323A) vector into the ATCC13032::hom(R407H)-lysC(L377K)-clpC_M4 of the above Example. Further, as a control thereof, a strain was prepared by introducing only ilvA(V323A) variation into ATCC13032::-hom(R407H)-lysC(L377K).

The prepared strains were cultured in the same manner as in the flask culture method of Example 4-1, and the concentrations of L-isoleucine in the cultures were analyzed. The analyzed concentrations of L-isoleucine in the cultures of the strains are as in Table 11 below.

**[Table 11]**

| | Strain | L-isoleucine (g/L) | | | |
|---|---|---|---|---|---|
| | | Batch 1 | Batch 2 | Batch 3 | Mean |
| Control group | ATCC13032::-hom(R407H)-lysC(L377K)/pECCG117-ilvA(V323A) | 4.1 | 4.0 | 4.3 | 4.13 |
| Experimental group | ATCC13032::hom(R407H)-lysC(L377K)-clpC/pECCG117-ilvA(V323A) | 6.3 | 5.7 | 5.6 | 5.87 |

As shown in Table 11, it was confirmed that, as compared to ATCC13032::-hom(R407H)-lysC(L377K)/pECCG117-ilvA(V323A), in which only ilvA(V323A) variation was introduced into ATCC13032::-hom(R407H)-lysC(L377K), ATCC13032::hom(R407H)-lysC(L377K)-clpC_M4/pECCG117-ilvA(V323A), in which clpC_M4 variation was further introduced, showed about 42% increase in the concentration of L-isoleucine. The above results indicate that the introduction of the clpC variation into the L-isoleucine producing strain of the genus *Corynebacterium* is effective in the production of L-isoleucine.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### Effect of the invention

A microorganism according to the present disclosure may produce branched-chain amino acids with high efficiency. Further, the produced branched-chain amino acids may be applied to various products, such as pharmaceutical raw materials and food additives, animal feeds, nutrients, pesticides, bactericides, etc.

## Claims

1. A microorganism of the genus *Corynebacterium,* wherein ATP-dependent protease activity is weakened, as compared to an unmodified microorganism.

2. The microorganism of the genus *Corynebacterium* of claim 1, wherein the microorganism has branched-chain amino acid producing ability.

3. The microorganism of the genus *Corynebacterium* of claim 2, wherein the branched-chain amino acid is L-valine or L-isoleucine.

4. The microorganism of the genus *Corynebacterium* of claim 1, wherein the weakening is weakening of the activity of ATP-dependent Clp protease ATP-binding subunit.

5. The microorganism of the genus *Corynebacterium* of claim 4, wherein the ATP-dependent Clp protease ATP-binding subunit is derived from the genus *Corynebacterium.*

6. The microorganism of the genus *Corynebacterium* of claim 4, wherein the ATP-dependent Clp protease ATP-binding subunit includes a polypeptide represented by SEQ ID NO: 5 or an amino acid sequence having 90% or more identity thereto.

7. The microorganism of the genus *Corynebacterium* of claim 1, wherein the microorganism includes a polypeptide having a deletion of amino acids corresponding to positions 431 to 433, based on a sequence represented by SEQ ID NO: 5, or a polynucleotide having a deletion of nucleotides corresponding to positions 1,291 to 1,299, based on a sequence represented by SEQ ID NO: 6.

8. The microorganism of the genus *Corynebacterium* of claim 1, wherein the microorganism of the genus *Corynebacterium* is *Corynebacterium glutamicum.*

9. A ClpC variant, wherein an amino acid sequence corresponding to positions 431 to 433 is deleted, based on an amino acid sequence represented by SEQ ID NO: 5.

10. The ClpC variant of claim 9, wherein the variant includes a polypeptide represented by an amino acid sequence of SEQ ID NO: 1 or an amino acid sequence having 90% or more identity thereto.

11. A polynucleotide encoding the ClpC variant of claim 9.

12. A method of producing branched-chain amino acids, the method comprising a step of culturing, in a medium, a microorganism of the genus *Corynebacterium,* in which ATP-dependent protease activity is weakened, as compared to an unmodified microorganism, or a microorganism including a ClpC variant, in which an amino acid sequence corresponding to positions 431 to 433 is deleted, based on an amino acid sequence represented by SEQ ID NO: 5, or a polynucleotide encoding the ClpC variant.

13. The method of claim 12, further comprising the step of recovering branched-chain amino acids from the medium or from the microorganism, after the culturing step.

14. Use of a microorganism of the genus *Corynebacterium,* in which ATP-dependent protease activity is weakened, as compared to an unmodified microorganism, in the production of branched-chain amino acids.

15. Use of a ClpC variant, in which an amino acid sequence corresponding to positions 431 to 433 is deleted, based on an amino acid sequence represented by SEQ ID NO: 5, or a polynucleotide encoding the ClpC variant, in the production of branched-chain amino acids.
